# FASCICULE DE BREVET EUROPEEN

(11) **EP 1 766 082 B1**
(45) Date de publication et mention de la délivrance du brevet: **01.08.2012**
(21) Numéro de dépôt: 05777306.1
(22) Date de dépôt: 14.06.2005
(51) Int. Cl.: C12Q 1/68, C07K 14/435

(54) **PROTEINE COG47 ET POLYMORPHISME DU GENE S100BETA**
COG47-PROTEIN UND S100BETA-GENPOLYMORPHISMUS
COG47 PROTEIN AND S100BETA GENE POLYMORPHISM

(30) Priorité: 14.06.2004 FR 0406428
(43) Date de publication de la demande: 28.03.2007
(73) Titulaire: Genoscreen, 59120 Loos (FR)
(72) Inventeur: AMOUYEL, Philippe, F-59700 Marcq en Baroeul (FR); LAMBERT, Jean-Charles, F-59200 Tourcoing (FR); FERREIRA, Stéphanie, F-59000 Lille (FR)
(74) Mandataire: Faivre Petit, Frédérique
(86) Numéro de dépôt international: PCT/FR2005/001480
(87) Numéro de publication internationale: WO 2006/000719

(56) Documents cités:
- DATABASE Geneseq [Online] 9 juillet 2003 (2003-07-09), "Human cancer related protein SEQ ID NO:360." XP002354089 extrait de EBI accession no. GSN:ABR58703 Database accession no. ABR58703
- AVRAMOPOULOS DIMITRIOS ET AL: "DNA polymorphisms in the 3' untranslated region of genes on human chromosome 21" GENOMICS, vol. 15, no. 1, 1993, pages 98-102, XP002307791 ISSN: 0888-7543
- PETZOLD A ET AL: "Cerebrospinal fluid S100B correlates with brain atrophy in Alzheimer's disease." NEUROSCIENCE LETTERS, vol. 336, no. 3, 23 janvier 2003 (2003-01-23), pages 167-170, XP002307792 ISSN: 0304-3940
- ALLORE R J ET AL: "CLONING AND EXPRESSION OF THE HUMAN S100-BETA GENE" JOURNAL OF BIOLOGICAL CHEMISTRY, vol. 265, no. 26, 1990, pages 15537-15543, XP002307793 ISSN: 0021-9258

### LISTE DE SEQUENCES

<110> Genoscreen
<120> Protéine Cog47 et polymorphisme du gène S100b
<130> D21744
<150> FR0406428
   <151> 2004-06-14
<160> 3
<170> PatentIn version 3.2
<210> 1
   <211> 94
   <212> PRT
   <213> Homo sapiens
<220>
   <223> Protéine Cog47
<400> 1
   Met Ser Glu Leu Glu Lys Ala Met Val Ala Leu Ile Asp Val Phe His
   1               5                   10                  15
   Gln Tyr Ser Gly Arg Glu Gly Asp Lys His Lys Leu Lys Lys Ser Glu
               20                  25                  30
   Leu Lys Glu Leu Ile Asn Asn Glu Leu Ser His Phe Leu Glu Arg Trp
           35                  40                  45
   Ser Leu Pro Met Leu Pro Arg Leu Val Ser Asn Phe Leu Cys Ser Ser
       50                  55                  60
   Tyr Pro Pro Ala Leu Ala Ser Gln Ser Ala Gly Ile Thr Gly Asn Gln
   65                  70                  75                  80
   Arg Ala Gly Gly Cys Gly Gln Ser His Gly Asn Thr Gly Gln
                   85                  90
<210> 2
   <211> 1189
   <212> ARN
   <213> Homo sapiens
<220>
   <223> ARNm de Cog47
<400> 2
   ugccgcccag gacccgcagc agagacgacg ccugcagcaa ggagaccagg aaggggugag     60
   acaaggaaga ggaugucuga gcuggagaag gccauggugg cccucaucga cguuuuccac    120
   caauauucug gaagggaggg agacaagcac aagcugaaga aauccgaacu caaggagcuc    180
   aucaacaaug agcuuuccca uuucuuagag agauggaguc ucccuauguu gcccaggcug    240
   gucucaaacu uccugugcuc aaguuauccu ccugccuuag cuucccaaag ugcugggauu    300
   acaggaaauc aaagagcagg agguugugga caaagucaug gaaacacugg acaaugaugg    360
   agacggcgaa ugugacuucc aggaauucau ggccuuuguu gccaugguua cuacugccug    420
   ccacgaguuc uuugaacaug agugagauua gaaagcagcc aaaccuuucc uguaacagag    480
   acggucaugc aagaaagcag acagcaaggg cuugcagccu aguaggagcu gagcuuucca    540
   gccguguugu agcuaauuag gaagcuugau uugcuuugug auugaaaaau ugaaaaccuc    600
   uuuccaaagg cuguuuuaac ggccugcauc auucuuucug cuauauuagg ccugugugua    660
   agcugacugg ccccagggac ucuuguuaac aguaacuuag gagucagguc ucagugauaa    720
   agcgugcacc gugcagcccg ccauggccgu guagacccua acccggaggg aacccugacu    780
   acagaaauua ccccggggca cccuuaaaac uuccacuacc uuuaaaaaac aaagccuuau    840
   ccagcauuau uugaaaacac ugcuguucuu uaaaugcguu ccucauccau gcagauaaca    900
   gcugguuggc cgguguggcc cugcaagggc gugguggcuu cggccugcuu cccgggaugc    960
   gccugaucac caggugaacg cucagcgcug gcagcguccu ggaaaaagca acuccaucag   1020
   aacucgcaau ccgagccagc ucugggggcu ccagcguggc cuccgugacc caugcgauuc    1080
   aagucgcggc ugcaggaucc uugccuccaa cgugccucca gcacaugcgg cuuccgaggg   1140
   cacuaccggg ggcucugagc caccgcgagg gccugcguuc aauaaaaag                1189
<210> 3
   <211> 1095
   <212> ARN
   <213> Homo sapiens
<220>
   <223> ARNm de S100-b normal
<400> 3
   ugccgcccag gacccgcagc agagacgacg ccugcagcaa ggagaccagg aaggggugag     60
   acaaggaaga ggaugucuga gcuggagaag gccauggugg cccucaucga cguuuuccac    120
   caauauucug gaagggaggg agacaagcac aagcugaaga aauccgaacu caaggagcuc    180
   aucaacaaug agcuuuccca uuucuuagag gaaaucaaag agcaggaggu uguggacaaa    240
   gucauggaaa cacuggacaa ugauggagac ggcgaaugug acuuccagga auucauggcc    300
   uuuguugcca ugguuacuac ugccugccac gaguucuuug aacaugagug agauuagaaa    360
   gcagccaaac cuuuccugua acagagacgg ucaugcaaga aagcagacag caagggcuug    420
   cagccuagua ggagcugagc uuuccagccg uguuguagcu aauuaggaag cuugauuugc    480
   uuugugauug aaaaauugaa aaccucuuuc caaaggcugu uuuaacggcc ugcaucauuc    540
   uuucugcuau auuaggccug uguguaagcu gacuggcccc agggacucuu guuaacagua    600
   acuuaggagu caggucucag ugauaaagcg ugcaccgugc agcccgccau ggccguguag    660
   acccuaaccc ggagggaacc cugacuacag aaauuacccc ggggcacccu uaaaacuucc    720
   acuaccuuua aaaaacaaag ccuuauccag cauuauuuga aaacacugcu guucuuuaaa    780
   ugcguuccuc auccaugcag auaacagcug guuggccggu guggcccugc aagggcgugg    840
   uggcuucggc cugcuucccg ggaugcgccu gaucaccagg ugaacgcuca gcgcuggcag    900
   cguccuggaa aaagcaacuc caucagaacu cgcaauccga gccagcucug ggggcuccag     960
   cguggccucc gugacccaug cgauucaagu cgcggcugca ggauccuugc cuccaacgug   1020
   ccuccagcac augcggcuuc cgagggcacu accgggggcu cugagccacc gcgagggccu   1080
   gcguucaaua aaaag                                                    1095

## Description

La présente invention concerne une protéine Cog⁴⁷ et son polynucléotide, associés notamment à une prédisposition au déclin cognitif, à une conversion vers une démence ou à une démence déjà existante, et le gène S100β présentant un polymorphisme.

L'invention concerne également une méthode de diagnostic et une trousse de diagnostic.

Les démences sont des maladies neurodégénératives du système nerveux central correspondant à un affaiblissement progressif de la mémoire et des autres fonctions cognitives. Après une période plus ou moins longue de détérioration des fonctions cognitives et de la personnalité, la maladie conduit à un état complet de dépendance.

Or, ces mêmes détériorations sont fréquemment observées à un niveau moindre lors du vieillissement cérébral dit "normal". Ainsi, pour certains auteurs, la démence apparaîtrait comme un processus inévitable au-delà d'un certain âge avec, au cours du vieillissement, un continuum entre déclin des fonctions cognitives et démence. La pathologie ne serait alors qu'une exagération ou qu'une accélération de ce vieillissement. Cette hypothèse du continuum est un des éléments clés qui justifie une approche globale ou parallèle de l'étude des déterminants du vieillissement cérébral normal et des pathologies cérébrales liées à l'âge. L'un des arguments principaux en faveur de cette hypothèse est la similarité entre des lésions neuropathologiques (plaques séniles et dégénérescence neurofibrillaires) observées fréquemment chez les personnes âgées sans détérioration cognitive et celles caractéristiques de la maladie d'Alzheimer, affection représentant près de 70% des démences. Cependant, la densité de ces lésions est toujours beaucoup plus importante et leur répartition plus large dans la maladie d'Alzheimer.

S'il existe un continuum entre détérioration des fonctions cognitives et démence, il est logique que des facteurs de risque génétique ou environnementaux soient commun à ces deux entités. L'implication du gène de l'apolipoprotéine E (APOE) à la fois comme déterminant génétique de la maladie d'Alzheimer et du niveau global des fonctions cognitives chez le sujet âgé, de même que de la présence de plaques séniles chez le patient atteint de maladie d'Alzheimer et chez le sujet âgé, va dans ce sens. Ceci suggère donc que la connaissance de certains facteurs génétiques puisse permettre à la fois une meilleure compréhension des déterminants du déclin cognitif chez la personne âgée et de l'apparition d'une démence. Cependant l'étude de tels marqueurs génétiques doit reposer sur une hypothèse physiophathologique forte.

Il est connu que l'activation des astrocytes contribue à la maintenance et à la sauvegarde du système nerveux central, en particulier suite à des événements aigus et limités dans le temps. Cependant, dans le cadre de lésions chroniques et/ou répétées induisant une activation systématique de ces astrocytes, ce mécanisme adaptatif et de réparation pourrait conduire à une progression plus importante et rapide d'une lésion ou d'une pathologie. Cette activation chronique apparaît au cours du vieillissement cérébral dit "normal" et prend une importance considérable lors de pathologies telles que la maladie d'Alzheimer. Ces simples observations suggèrent donc que les facteurs contrôlant le développement d'une activation astrocytaire chronique pourraient jouer un rôle angulaire entre détérioration des fonctions cognitives, observables aussi au cours du vieillissement, et démence.

Parmi les facteurs potentiels contrôlant cette réponse astrocytaire, la protéine S100β semble être une cible privilégiée pour de nombreuses raisons. Au niveau physiologique, S100β, majoritairement produit par les astrocytes, apparaît être impliquée au niveau du développement et de la maintenance du cerveau mais aussi au niveau des processus cognitifs et de leur mise en place. Néanmoins, son importance par rapport à ces processus est toujours mal définie et pourrait même sembler contradictoire. En effet, l'injection d'anticorps dirigés contre S100β dans le cerveau de rats, conduit à des troubles de l'apprentissage et de la mémoire alors que des souris transgéniques surexprimant S100β présentent quant à elles des troubles du comportement proches des troubles que l'on peut observer pour certaines démences. Cette apparence contradictoire pourrait s'expliquer par le fait que S100β est neuroprotecteur pour des concentrations de l'ordre du nanomolaire et neurotoxiques pour des concentrations de l'ordre du micromolaire. Au niveau pathologique, cette dernière observation pourrait être essentielle. En effet, les astrocytes activés au cours de la maladie d'Alzheimer surexpriment de façon marquée S100β. Cette protéine est capable, par action autocrine, de promouvoir la prolifération et l'hypertrophie astrocytaire. Or, de façon intéressante, la plupart des astrocytes activés et surexprimant S100β sont fortement associés à la présence de plaques amyloïdes, l'un des deux marqueurs pathologiques de la maladie d'Alzheimer.

De façon moins spécifique, cette surexpression pourrait être aussi un marqueur de l'apparition de troubles cognitifs ou de dommages cérébraux. Ainsi, une surexpression de cette protéine est un indicateur de risque de dommages cérébraux chez le foetus ou le nouveau-né. De même, une concentration élevée en S100β est un indicateur pronostique d'une plus grande difficulté de récupération suite à un traumatisme crânien ou d'un risque plus important d'apparitions de troubles cognitifs légers à sévères après acte chirurgical.

Ces données indiquent donc que la protéine S100β serait importante dans l'établissement des fonctions cognitives et de leur détérioration potentielle. De fait, toutes modifications susceptibles de moduler les fonctions biologiques de cette protéine pourraient être essentielles pour définir les risques de déclins cognitifs, voir de démence chez la personne âgée mais aussi par exemple après acte chirurgical.

Jusqu'à présent, les outils utilisés pour caractériser un déclin cognitif, une conversion vers une démence ou une démence déjà existante sont basés sur des tests psychométriques très pointus dont l'utilisation est réservée aux praticiens. Cependant, 10% à 37% des diagnostics s'avèrent inexacts. De plus, ils sont relativement peu efficaces dans les premières étapes de la pathologie en raison de l'absence de signes cliniques objectifs et mesurables.

Ainsi, un certain nombre de tests biologiques, qu'ils soient génétiques ou protéomiques, ont été élaborés pour palier ces déficiences.

Il a été proposé que l'allèle ε4 du gène de l'APOE puisse servir dans ce cadre. Cependant, il a été depuis démontré que ce marqueur génétique avait une faible valeur prédictive au niveau du déclin cognitif ou de la conversion vers une démence.

Au niveau protéique, une quantification des protéines Tau et peptides amyloïdes a été mise en place à partir d'échantillons de sérum ou de liquide céphalo-rachidien. Cependant, les résultats obtenus ont montré que ces mesures biologiques n'étaient pas plus efficaces que les tests psychométriques, même s'ils permettaient d'orienter le diagnostic.

La maladie d'Alzheimer et plus généralement les démences constituent un problème majeur de santé publique dont l'importance s'accroît avec le vieillissement des populations humaines.

Dans l'état actuel de nos connaissances, il n'existe pas de traitement curatif de cette maladie. L'évolution se fait vers une désocialisation massive liée à la désorientation temporo-spatiale et aux troubles de mémoire incompatibles avec l'autonomie nécessaire à une vie sociale autonome. Le coût d'une telle affection en matière de prise en charge et de dépendance s'avère considérable et peut, à terme, engorger le système de soins et de prise en charge.

Il existe aujourd'hui cependant quelques traitements symptomatiques fondés sur une amélioration des fonctions cognitives et de la mémoire agissant sur certains neurotransmetteurs. Ces médicaments font partie de la classe des inhibiteurs de l'acétylcholinestérase qui prolongent l'action de l'acétylcholine qui est un neurotransmetteur essentiel. Quatre molécules sont aujourd'hui disponibles : la Tacrine^{R} (laboratoires OTL Pharma), le Donezepil^{R} (laboratoires Eisai), la Rivastigmine^{R} (laboratoires Novartis), la Galantamine^{R} (laboratoires Janssen Cilag). Par ailleurs, il a été récemment proposé que les statines, molécules initialement développées pour le traitement de l'hypercholestérolémie, puissent aussi être utilisées. Enfin, une nouvelle molécule, la Mémantine (laboratoires Lundbeck), un antagoniste du récepteur NMDA à action anti-glutamatergique est également protégé.

Ces traitements, lorsque les patients peuvent en bénéficier, permettent de retarder, dans une certaine mesure, la désocialisation. Or, différents travaux ont permis de montrer que même en l'absence de traitement curatif, le simple fait de retarder de 5 ans l'apparition des signes cliniques de la maladie d'Alzheimer permettrait de diminuer de moitié le nombre de cas prévalents de cette pathologie, essentiellement en raison de la mortalité concurrentielle importante à ces âges avancés.

Les traitements sympthomatiques de la maladie d'Alzheimer n'ont d'indication que dans les formes de démences légères et modérées. En effet, un certain potentiel neuronal doit encore être présent pour que ces médications agissent. Aussi, afin que les patients puissent bénéficier au mieux de ces traitements, une identification précoce des patients qui présentent des signes discrets de démence est indispensable.

Depuis le milieu des années 1990, le développent des inhibiteurs des cholinestérases a ouvert la voie à une prise en charge thérapeutique des malades présentant des formes légères à modérées de la maladie d'Alzheimer. Actuellement, plusieurs essais thérapeutiques sont en cours sur ce que l'on appelle le "Mild Cognitive Impairement". Cette atteinte intellectuelle modérée, fondée sur des tests des fonctions cognitives, est encore imprécise et le risque de traiter à tort des patients non atteints de démence et n'ayant que des troubles mineurs, liés par exemple à un épisode dépressif, est important.

De nombreux travaux montrent que la quantification de S100β au niveau plasmatique est un très bon indicateur de l'importance de lésions cérébrales post-traumatiques que ce soit chez l'enfant ou l'adulte. Cette quantification est un très bon marqueur du niveau et du temps de récupération ainsi que des risques de complications liés à ce type de pathologie. Par exemple, monitorer la concentration de S100β est proposé pour prédire le développement d'une surpression intracrânienne secondaire suite à un traumatisme crânien, cette complication étant potentiellement fatale.

De même, la concentration en S100β, de façon plus spécifique, est associée au niveau d'altération de la barrière hémato-encéphalique, de la présence de tumeurs cérébrales ou de métastases. D'ailleurs, le niveau de S100β corrèle avec la taille des tumeurs et pourrait donc être utilisé comme marqueur précoce des tumeurs cérébrales.

Enfin, le taux de S100β est un marqueur très précoce de lésions cérébrales chez le foetus ou le nouveau-né, alors que les méthodes standard d'évaluation ne permettent pas de détecter ce type de lésions. Il a donc été proposé que S100β puisse être utilisé comme marqueur des lésions cérébrales en médecine périnatale.

La quantité de S100β est aussi un marqueur sensible de risques de complications après opération cardiaque. Ainsi, cette quantification est prédictive du risque de survenue d'une attaque cérébrale, cette pathologie étant un problème récurrent après opération à coeur ouvert. Elle est aussi corrélée à l'importance de cette attaque et donc au risque de décès du patient ayant subi une telle complication. Par ailleurs, la concentration en S100β est un indicateur d'un déclin cognitif suite à ce type d'opération et du niveau de récupération de ces fonctions cognitives.

Finalement, la mesure du taux de S100β est un excellent marqueur du succès de la lyse d'un caillot après attaque cérébrale.

Il a déjà été décrit que S100β pouvait être utilisé comme marqueur de tumeurs cérébrales. Cependant, les évolutions d'autres lésions cancéreuses peuvent être monitorées par la mesure de S100β circulant. Ainsi cette mesure apparaît comme la plus appropriée pour suivre l'apparition et l'évolution de métastases provenant de mélanomes.

Le brevet US 6/555,327 divulgue une méthode de détermination quantitative de la protéine S100β et S100ββ pour diagnostiquer des traumatismes du cerveau, les complications neurologiques après pontage et les mélanomes malins.

La demande de brevet internationale WO 00/26668 divulgue une méthode pour diagnostiquer et pronostiquer un cancer chez un sujet détectant au moins une protéine S 100 parmi S100AG, S100A7, S100A8, S100A9, à l'aide d'un test immunologique.

La demande de brevet internationale WO98/01471 divulgue un peptide contenant au moins un fragment de la protéine S100β. Il est également divulgué une méthode de détermination de la présence dudit peptide dans un échantillon pour diagnostiquer de manière générale des maladies neurologiques.

La demande de brevet internationale WO 02/089656 divulgue une méthode pour diagnostiquer un événement clinique dans des tissus de patients, plus particulièrement l'infarctus du myocarde, par la détection de la protéine S100β.

Le brevet US 5/849,528 divulgue deux protéines humaines S100(S100T1 et S100T2). Le document divulgue également un polynucléotide codant pour ladite protéine, ainsi qu'une composition contenant ce polynucléotide pour diagnostiquer la progression des changements neuropathologiques dans la maladie d'Alzheimer.

Enfin, la demande de brevet US 2002/160,425 divulgue principalement une méthode de diagnostic et de différenciation de la démence liée à Alzheimer chez un mammifère, en utilisant un anticorps liant spécifiquement un marqueur biochimique de la maladie d'Alzheimer, tel que la protéine S100β, et en déterminant la présence de ce marqueur.

Les méthodes de diagnostic décrites ci-dessus, en l'occurrence pour la maladie d'Alzheimer, ne permettent pas de diagnostiquer suffisamment tôt la maladie pour qu'un traitement adapté puisse être réellement efficace à long terme.

Le besoin d'un outil diagnostique simple de routine pouvant être réalisé sur sang circulant permettant de renforcer la certitude diagnostique et de garantir les indications thérapeutiques avant l'apparition par exemple des symptômes avancés de la maladie d'Alzheimer se fait donc ressentir.

Les inventeurs se sont donc donnés pour but de mettre au point un outil diagnostique simple de routine pouvant être réalisé sur sang circulant et permettant de diagnostiquer un marqueur biologique du déclin cognitif, d'une conversion vers une démence ou une démence déjà existante.

La présente invention a ainsi pour objet une protéine dénommée Cog⁴⁷ représentée par la séquence SEQ ID N°1 présentant une séquence d'acides aminés différente de celle de la protéine S100β normale.

Il est démontré par les exemples qui suivent que la protéine Cog⁴⁷ est associée notamment à une prédisposition au déclin cognitif, à une conversion vers une démence ou à une démence déjà existante. Ces exemples ne doivent pas exclure que la protéine Cog⁴⁷ puisse être impliquée dans d'autres pathologies.

Dans la présente invention, l'expression "déclin cognitif', signifie la perte progressive non pathologique des fonctions mnésiques et cognitives supérieures.

Dans la présente invention, l'expression "conversion vers une démence", signifie l'apparition d'événement correspondant à l'apparition clinique d'une démence, d'un état asymptomatique ou infra-clinique à un état symptomatique ou pathologique.

Dans la présente invention, l'expression "démence déjà existante", signifie cas prévalent de démence à l'inclusion dans l'étude ou au moment du diagnostic.

La présente invention a encore pour objet un anticorps polyclonal ou monoclonal, ou fragment dudit anticorps, se fixant de manière spécifique sur la protéine Cog⁴⁷ par affinité avec la séquence d'acides aminés différente de celle de la protéine S100β normale et l'utilisation dudit anticorps polyclonal ou monoclonal, ou fragment dudit anticorps pour la mise en oeuvre d'un test immunologique *in vitro* chez l'humain.

Dans la présente invention, par "affinité avec la séquence" on entend une reconnaissance par l'anticorps de la séquence d'acides aminés, soit par reconnaissance de l'alignement desdits acides aminés, soit par reconnaissance de la conformation dans l'espace desdits acides aminés.

Dans la présente invention, l'expression "fragment dudit anticorps" signifie partie dudit anticorps permettant au moins la reconnaissance de l'épitope de la protéine Cog⁴⁷, ledit épitope étant présent au niveau de la séquence d'acides aminés différente de celle de la protéine S100β normale. Le fragment contient au minimum le ou les paratopes correspondants.

La présente invention a encore pour objet l'utilisation d'une protéine dénommée Cog⁴⁷ telle que définie précédemment pour produire *in vitro* des anticorps se fixant de manière spécifique sur ladite protéine.

La présente invention a en outre pour objet un procédé pour déterminer la présence d'une protéine Cog⁴⁷ représentée par la séquence SEQ ID N°1 dans un échantillon humain comprenant les étapes de :
- laisser l'échantillon à analyser réagir avec un premier anticorps tel que décrit précédemment,
- laisser l'échantillon à analyser réagir avec un second anticorps dirigé spécifiquement contre l'anticorps tel que décrit précédemment,
- laver, et
- détecter la quantité de protéine Cog⁴⁷ dans l'échantillon.

De préférence, dans le procédé décrit précédemment l'anticorps est un anticorps monoclonal.

Par échantillon humain, on entend désigner tout fluide ou tissu corporel prélevé chez l'homme, comme par exemple un prélèvement sanguin ou céphalo-rachidien.

Un autre objet de l'invention concerne le polynucléotide ARNm isolé et purifié codant pour la protéine Cog⁴⁷ représenté par la séquence SEQ ID N°2.

Encore un autre objet de l'invention concerne un polynucléotide ARNm d'interférence ciblant de manière spécifique l'ARNm décrit précédemment ainsi qu'un polynucléotide ADN capable d'exprimer ledit polynucléotide ARNm d'interférence. L'invention concerne également ledit polynucléotide ARNm d'interférence ou polynucléotide ADN selon l'invention à usage thérapeutique pour inhiber ou diminuer l'expression de la protéine Cog⁴⁷.

Par "usage thérapeutique", on entend dans la présente invention "utilisation en tant que médicament".

Les indications thérapeutiques sont principalement le déclin cognitif, la conversion vers une démence et la démence déjà existante.

La technologie d'ARN interférent (RNAi) permet d'éteindre un gène dans une cellule et offre de nombreuses applications en thérapeutique.

L'ARN interférent peut être obtenu soit par l'administration d'ARN double brin qui est dégradé dans la cellule en petits ARN interférents ou siRNA ("small interfering RNA"), soit par l'administration d'ADN double brin (ddRNAi pour "DNA directed RNA interference") qui déclenche la production des siRNA. Cette technologie est suffisamment décrite dans la littérature scientifique et fait l'objet de dépôts de demandes de brevets (sociétés SIRNA THERAPEUTICS, BENITEC,...)

La présente invention a en outre pour objet un vecteur d'expression de la protéine Cog⁴⁷ comprenant au moins la séquence ADN SEQ ID N°1 et les éléments permettant l'expression de ladite protéine dans une cellule hôte procaryote dans le but de produire une protéine recombinante.

La présente invention a encore pour objet une trousse de diagnostic pour déterminer la présence de la protéine Cog⁴⁷ représentée par la séquence SEQ ID N°1 dans un échantillon humain, comprenant une protéine Cog⁴⁷ telle que décrite précédemment et/ou des anticorps polyclonaux ou monoclonaux, ou leurs fragments, tels que décrits précédemment.

Un autre objet concerne l'utilisation du polynucléotide ARNm tel que décrit précédemment, ou un de ses fragments, pour diagnostiquer *in vitro* une prédisposition au déclin cognitif, la conversion vers une démence et la démence déjà existante.

Un autre objet de l'invention concerne une souris transgénique comprenant dans son génome la séquence nucléotidique nécessaire pour exprimer la protéine Cog⁴⁷ selon la séquence SEQ ID N°1 dans le but de développer un modèle d'étude de l'implication de Cog⁴⁷ dans des processus pathologiques et de cibles thérapeutiques potentielles

La présente divulgation a également pour objet un gène S100β humain isolé représenté par la séquence SEQ ID N°3, caractérisé en ce que ledit gène présente au moins un polymorphisme associé à une prédisposition au déclin cognitif, à une conversion vers une démence ou à une démence déjà existante.

Il est effectivement démontré par les exemples qui suivent que certains polymorphismes sur le gène S100β humain sont associés à une prédisposition au déclin cognitif, à une conversion vers une démence ou à une démence déjà existante.

Dans un mode de réalisation très préférentiel selon l'invention, le polymorphisme du gène S100β est caractérisé en ce que le polymorphisme se situe au niveau du nucléotide thymidine - 100 par rapport au site d'initiation de transcription dudit gène S100β. De préférence encore, le polymorphisme du gène est caractérisé en ce que le nucléotide thymidine est remplacé par un nucléotide cytosine.

La présente divulgation a également pour objet l'utilisation du gène S100β humain tel que défini précédemment, ou un fragment de celui-ci, pour diagnostiquer une prédisposition au déclin cognitif, une conversion vers une démence ou une démence déjà existante.

La présente divulgation a en outre pour objet l'utilisation du gène S100β humain représenté par la séquence SED ID N°3, ou un fragment de celui-ci, pour diagnostiquer une prédisposition au déclin cognitif, une conversion vers une démence ou une démence déjà existante, caractérisée en ce que le polymorphisme se situe au niveau du nucléotide +2766 par rapport au site d'initiation de transcription dudit gène.

Dans la présente invention, l'expression "un fragment de celui-ci" associé à "gène" signifie une partie dudit gène.

Dans un mode de réalisation préférentiel, dans l'utilisation du gène S100β décrit précédemment, le nucléotide guanidine est remplacé par un nucléotide cytosine.

La présente divulgation a également pour objet l'utilisation du gène S100β humain représenté par la séquence SED ID N°3, ou un fragment de celui-ci, pour diagnostiquer une prédisposition au déclin cognitif, une conversion vers une démence ou un démence déjà existante, caractérisée en ce que le polymorphisme se situe au niveau du nucléotide adénine + 2963 par rapport au site d'initiation de transcription dudit gène.

Dans un mode de réalisation préférentiel, dans l'utilisation du gène S100β décrit précédemment, le nucléotide guadinine est remplacé par un nucléotide adénine.

La présente divulgation a encore pour objet l'utilisation du gène S100β humain représenté par la séquence SED ID N°3, ou un fragment de celui-ci, pour diagnostiquer une prédisposition au déclin cognitif, une conversion vers une démence ou un démence déjà existante, caractérisée en ce que le polymorphisme se situe au niveau du nucléotide guanidine + 3942 par rapport au site d'initiation de transcription dudit gène.

Dans un mode de réalisation préférentiel, dans l'utilisation du gène S100β décrit précédemment, le nucléotide guanidine est remplacé par un nucléotide adénine.

Un autre objet concerne une trousse de diagnostic pour déterminer la présence d'un polymorphisme du gène S100β représenté par la séquence SEQ ID N°3 associé à une prédisposition au déclin cognitif, à une conversion vers une démence ou à une démence déjà existante, dans un échantillon comprenant un ou plusieurs oligonucléotides de 20 à 25 pb complémentaire des polymorphismes tels que décrits précédemment.

La présente divulgation a encore pour objet une méthode de diagnostic *in vitro* de la prédisposition au déclin cognitif, à une conversion vers une démence ou à une démence déjà existante chez un humain comprenant la détermination de la présence d'un polymorphisme tel que décrit précédemment et/ou des protéines associées audit polymorphisme, notamment la protéine Cog⁴⁷.

Selon un dernier aspect, la présente invention a pour objet un procédé d'identification *in vitro* d'une molécule capable d'interagir avec la protéine Cog⁴⁷ comprenant les étapes suivantes :
a) mettre en présence un modèle cellulaire approprié avec la protéine Cog⁴⁷ et ladite molécule à identifier pour caractériser la quantité de protéines Tau phosphorylées à l'aide d'au moins un marqueur spécifique de phosphorylation de la protéine Tau, et/ou
b) mettre en présence un modèle cellulaire approprié avec la protéine Cog⁴⁷ et ladite molécule à identifier pour caractériser le niveau d'expression d'au moins un marqueur d'inflammation de la microglie,
c) lorsque :
   - la quantité de protéines Tau phosphorylées caractérisée à l'étape a) est inférieure à la quantité de protéines Tau phosphorylées caractérisée en l'absence de ladite molécule, et/ou
   - le niveau d'expression dudit au moins un marqueur d'inflammation de la microglie caractérisé à l'étape b) est inférieur au niveau d'expression dudit marqueur caractérisé en l'absence de ladite molécule,
   identifier ladite molécule comme étant capable d'interagir avec la protéine Cog⁴⁷.

La molécule à identifier capable de moduler l'activité de la protéine Cog⁴⁷ peut être une molécule chimique ou biologique (isolée, recombinante ou synthétique).

Le modèle cellulaire approprié pour caractériser la quantité de protéines Tau phosphorylées est bien connu de l'homme de l'art et est en général un modèle cellulaire neuronal.

Le modèle cellulaire approprié pour caractériser le niveau d'expression dudit au moins un marqueur de l'inflammation de la microglie est en général un modèle constitué par des cellules de la microglie.

Toutefois, l'homme de l'art peut envisager de modifier ces deux modèles cellulaires, voire de les combiner afin de caractériser la quantité de protéines Tau phosphorylées et le niveau d'expression du marqueur d'inflammation de la microglie de manière simultanée.

Chacun de ces modèles cellulaires est mis en présence avec la protéine Cog⁴⁷ et la molécule à identifier.

Lorsqu'il s'agit du modèle cellulaire neuronal utilisé pour caractériser la quantité de protéines Tau phosphorylées, la mise en présence de la protéine Cog⁴⁷ consiste en général en l'addition d'une solution de cette protéine Cog⁴⁷ audit modèle cellulaire.

Lorsque le modèle cellulaire utilisé pour caractériser le niveau d'expression du marqueur d'inflammation de la microglie est un modèle constitué par des cellules de la microglie, on préfère en général transformer dans une étape préliminaire ces cellules de la microglie à l'aide d'un acide nucléique codant pour la protéine Cog⁴⁷.

Selon un mode préféré de réalisation, ledit au moins un marqueur spécifique de phosphorylation de la protéine Tau est un anticorps dirigé contre un épitope phosphorylé de la protéine Tau, cet épitope étant caractéristique d'une dégénérescence neuronale. De préférence, au moins deux, trois ou quatre marqueurs spécifiques de phosphorylation sont utilisés.

D'autres marqueurs spécifiques de ladite phosphorylation bien connus de l'homme de l'art pourront être utilisés. Comme exemple d'un anticorps dirigé contre un épitope phosphorylé de la protéine Tau, on peut citer, sans toutefois s'y limiter, les anticorps monoclonaux AD2 et AT8.

Selon un autre mode préféré de réalisation, ledit au moins un marqueur d'inflammation de la microglie est une cytokine.

De manière préférée, on caractérise le niveau d'expression d'au moins deux, de préférence au moins trois, quatre, ou au moins cinq cytokines.

Comme exemple de cytokine, on peut citer, sans toutefois s'y limiter, le facteur de nécrose des tumeurs α (TNFα) et l'interleukine (IL6).

D'une manière optionnelle, la morphologie des cellules de la microglie est caractérisée en complément de la caractérisation du niveau d'expression dudit au moins un marqueur d'inflammation.

Selon une autre alternative, l'inflammation de la microglie peut être caractérisée au niveau tissulaire à l'aide d'un marquage à la ferritine.

Outre les dispositions qui précèdent, la présente invention comprend encore d'autres dispositions qui ressortiront du complément de description qui suit, qui se rapportent à l'exemple, ainsi qu'aux dessins annexés dans lesquels :
- La figure 1 illustre en 1(a) la séquence de l'ARNm et de la protéine correspondante de l'isoforme S100β normale, en 1(b) la séquence de l'ARNm et de la protéine correspondante de la séquence de l'isoforme Cog⁴⁷ à partir d'ARNm total ;
- La figure 2 illustre en 2(a) l'amplification par RT-PCR des ARNm correspondant à S100β normal et Cog⁴⁷ à partir de macrophages et lymphocytes humains et de lymphocytes de cercopithèque de Hylan ; la figure 2(b) illustre la comparaison inter-espèce des séquences protéiques de S100β normal et Cog⁴⁷ (après séquençage des ADN génomiques). En gris sont indiqués les acides aminés qui diffèrent entre les espèces étudiées ;
- La figure 3 illustre les niveaux d'expression des isoformes de S100β et Cog⁴⁷ dans le tissu cérébral de 85 patients souffrant de maladie d'Alzheimer et de 90 témoins ;
- La figure 4 illustre la corrélation entre le niveau d'expression de S100β et Cog⁴⁷ et le niveau de dégénérescence neurofibrillaire ;
- La figure 5 illustre la corrélation entre le niveau d'expression de S100β et Cog⁴⁷ et l'inflammation de la microglie.

Les exemples qui suivent permettent d'illustrer le meilleur mode de réalisation de l'invention, mais ils ne doivent pas pour autant être considérés comme réduisant la portée des revendications.

### Exemple : Etude du polymorphisme du gène S100β et de la protéine Cog⁴⁷ associée à une prédisposition au déclin cognitif, à une conversion vers une démence ou à une démence déjà existante

### 1. Matériel et méthodes :

### Population d'Eldnord

L'étude d'ELDNOR (ELDerly dans le NORd de La France) a été conduite dans le département du Nord, une région administrative de la France, entre mars et septembre 1993. Un échantillon aléatoire de 1055 individus des deux genres ayant entre 60 et 105 ans a été recruté dans des maisons de retraite. Un seul médecin qualifié a exécuté l'entrevue et le prélèvement de sang. Au début de l'entrevue, tous les sujets ont reçu une description orale de l'étude et ont signé un consentement en connaissance de cause. Pour ceux qui étaient mentalement incompétents, nous avons approché leur descendance ou leurs précepteurs juridiques. Le Comité d'éthique de l'université locale a approuvé l'étude. Un MMS a été réalisé pour tous les sujets. 187 individus ont été diagnostiqués avec une démence.

### Population de Limeil Brévannes

125 cas de démence (28,7% hommes, 85,5 ans) ont été recrutés dans le département long séjour de gérontologie de l'hôpital de Limeil Brévannes (France). La démence a été identifiée selon les critères de DSM-III-R. 36 individus présentant un déclin cognitif sans critères de démence ont été également recrutés. Des cerveaux (n = 167, 50,9% hommes, 79,6 ans) venant d'autopsies courantes effectuées aux Hospices Civils de Strasbourg (Est de la France) ont été utilisés comme témoins pour cette étude. Le recrutement a été conçu afin d'exclure des cas de démence et des patients souffrant de pathologies neurologiques. La plupart des sujets ont été admis moins de 48 heures avant la mort dans des services d'urgence et vivaient à domicile avant leur admission. Toutes les études ont été approuvées par les Comités d'éthique locaux.

### Population Alzheimer

Les échantillons témoins et Alzheimer sont Caucasiens, recrutés dans le nord de la France (cas Alzheimer: n = 589, âge = 72,3 ans, âge au début = 69,4 ans, 39,5% d'hommes; témoins : n = 663, l'âge = 72,5 ± 7,9 ans, 36% d'hommes). Le diagnostic probable de maladie d'Alzheimer a été établi selon les critères du DSM-III-R et du NINCDS-ADRDA. Les témoins caucasiens ont été définis comme sujets sans critères de démence de DMS-III-R et avec l'intégrité de leurs fonctions cognitives. Chaque individu ou apparenté a donné un consentement en connaissance de cause.

### Échantillons de cerveaux

Les 85 cerveaux de patients souffrant de maladie d'Alzheimer ont été obtenus à partir d'une banque de 114 patients atteints de formes définitives de la pathologie, et recrutés à Manchester et sa proche banlieue (Royaume Uni) durant les années 1986-2001 (âge moyen à la mort = 73,1 ± 9,1 ans ; âge moyen de début = 65,9 ± 10,3 ans; 51% de mâle). Tous les diagnostics ont été faits selon des critères neuropathologiques du CERAD.

La quantité de plaques amyloïdes Aβₙ₋₄₀, Aβₙ₋₄₂₍₄₃₎ et Aβ total (Aβₙ₋₄₀ + Aβ ₙ₋₄₂₍₄₃₎) a été mesurée par analyse d'image dans le cortex frontal (régions 8/9 de Brodmann) comme précédemment décrit. La charge de Tau a été déterminée dans 86 échantillons par un procédé standard utilisant l'anticorps monoclonal A T8 (Innogenetics, Belgique) en tant qu'anticorps primaire. La recherche de ferritine, comme marqueur de la microglie activé, a été réalisée sur 72 cerveaux en utilisant un procédé standard, une incubation avec un anticorps primaire contre la ferritine (sigma, R-V) ayant été utilisé durant la nuit à 4°C (à une dilution de 1:750). L'immunoréactivité à la ferritine a alors été visualisée par l'ajout de diaminobenzidène 3.3 (DAB).

Les 90 cerveaux de témoins (de 79,4 ± 6,1 ans; 43% d'hommes) proviennent d'un premier ensemble de 190 cerveaux obtenus à partir des autopsies courantes effectuées aux Hospices Civils de Strasbourg (France). Le recrutement a été conçu pour exclure des cas de démence. Les individus ont été recrutés dans un hôpital général et non pas au sein d'établissements médicaux gériatriques dans lesquels une majorité de patients peuvent présenter une démence. La plupart des cas ont été admis moins de 48 heures avant la mort dans des services d'urgence et vivaient à domicile avant leur admission. Les cas dont l'autopsie a visé des pathologies neurologiques ont été exclus. Les critères de Neuropathologie ont été appliqués pour définir des étapes de Braak et étaient conformes aux critères neuropathologique du CERAD.

### Séquençage et génotypage

Tous les exons, liaisons d'intron/exon ainsi que le promoteur proximal et l'intron 3 du gène S100β ont été examinés dans 32 témoins pour mettre en évidence des variations de séquence en utilisant l'analyse par D-HPLC (chromatographie liquide à haute performance dénaturante). La D-HPLC également connue sous le nom d'analyse hétéro-duplex modulée par température est une technique extrêmement sensible pour détecter une variation des séquences d'ADN, en particulier les substitutions de simples bases. Tous les variants identifiés par D-HPLC ont été confirmés par séquençage. Des produits de PCR ont été directement séquencés à l'aide du kit de séquençage Terminator colorant Taq (biosystèmes de Perkin Elmer, Foster City, CA).

Le génotype rs2000403 a été déterminé par amplification à partir d'un fragment à l'aide des amorces 5'-actctgaaccattcacggtg-3' et 5'-gtctctcaccaagccctatt-3'. Le génotype a alors été déterminé par digestion enzymatique (*NI*α III).

### RT-PCR en temps réel

L'ARN total des cerveaux a été extrait à partir de tissu congelé en utilisant un protocole basé sur l'utilisation de phénol/chloroforme (réactif TRIzol®; Invitrogen). La qualité de l'ARN total a été évaluée en utilisant le bioanalyseur 2100 d'Agilent et le rapport de l'ARN ribosomal 28S/18S a été systématiquement estimé (gamme de 0,0 à 1,8). La retrotranscription puis l'amplification PCR en temps réel a été exécutée à partir de 30 ng d'ADNc/ARN en utilisant la technologie Taqman, permettant ainsi de co-amplifier l'ADNc représentatif des gènes S100β, Cog⁴⁷, et de la β-actine comme décrit par le fournisseur (Applied biosystèmes).

### Analyses statistiques

L'analyse statistique a été réalisée en utilisant le logiciel statistique de SAS, v. 7.0 (SAS Institute Inc., Cary, NC). Le niveau de la dégradation totale d'ARN (estimée par le rapport d'ARNr 28S/18S) n'a pas été identifié en tant que facteur de confusion pour les niveaux d'expression mesuré de S100β ou Cog⁴⁷ (en utilisant un modèle de régression linéaire). Cependant, ce niveau de dégradation a été tout de même inclus dans les analyses multivariées de la covariance en utilisant un modèle linéaire général pour la comparaison de la quantité d'ARNm entre les cas d'Alzheimer et les témoins.

La charge de Tau, le niveau d'activation de la microglie et les quantifications des ARNm ont été log-transformés pour obtenir une distribution normalisée. L'effet du polymorphisme rs2000403 sur le niveau d'ARNm a été examiné en utilisant le test non paramétrique de Wilcoxon. Les ORs (permettant d'évaluer un risque de maladie d'Alzheimer pour un niveau élevé ou bas d'expression de Cog⁴⁷ ou de S100β) ont été évalués par régression logistique, les sous-populations ayant été définies en utilisant la médiane du niveau d'ARNm.

Les analyses univariées ont été exécutées en utilisant le test χ² de Pearson. Dans les analyses multivariées, nous avons codé le génotype du polymorphisme rs2000403 en dichotomisant les génotypes en fonction de la présence ou l'absence d'un allèle A (GG contre AA+GA). L'effet des ces variables sur la maladie a été évalué par un modèle logistique de régression linéaire ajusté sur l'âge et le sexe. Des coefficients de déséquilibre de liaison ont été estimés par paires dans les échantillons témoins. Des fréquences étendues d'haplotype des deux marqueurs ont été estimées en utilisant l'algorithme de dénombrement d'haplotype Myriad décrit par McLean et al.

### 2. Etude du polymorphisme du gène S100β et de protéine Cog⁴⁷ :

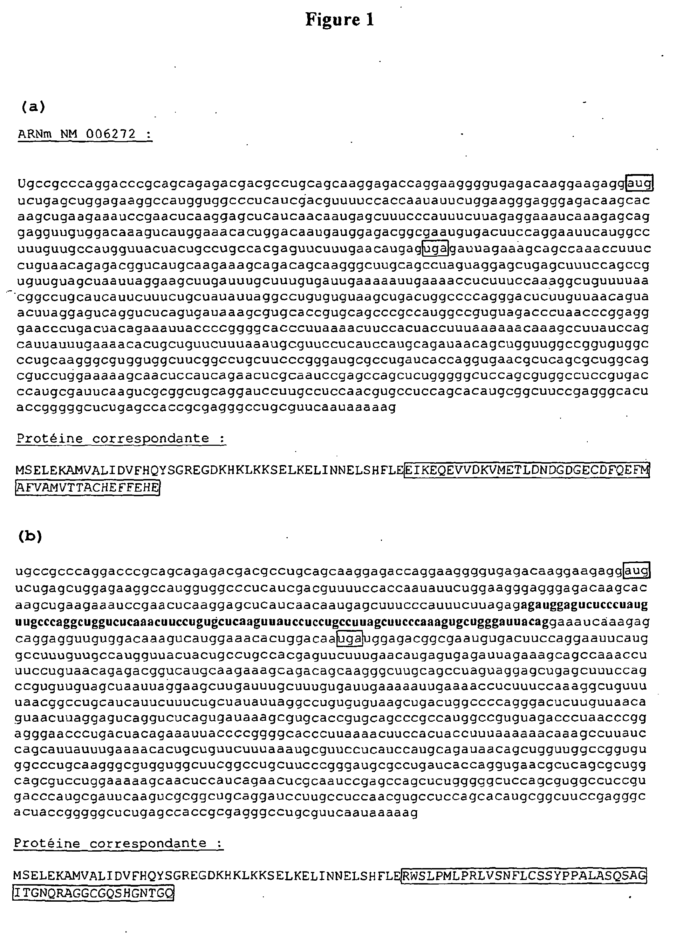

Par comparaison des séquences d'ESTs disponibles sur le site de l'université de Santa-Cruz (http://genome.ucsc.edu), il a été mis en évidence l'existence d'un exon inconnu dans le gène S100β constitué jusqu'à présent de 3 exons. Par RT-PCR, un 4^{ième} exon permettant d'établir une nouvelle séquence d'ARNm (Figure 1b) a été mis en évidence.

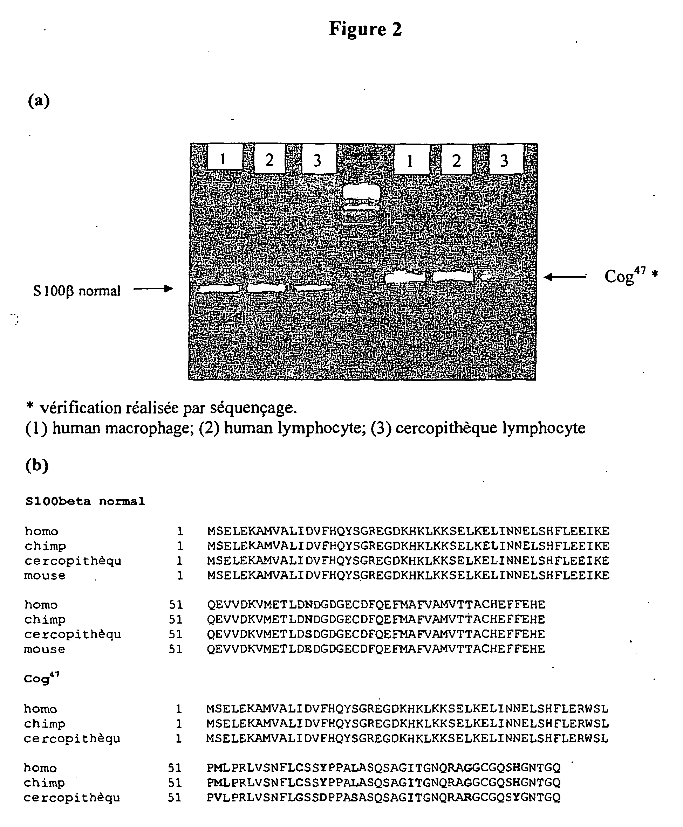

La nouvelle isoforme dénommée Cog⁴⁷ diffère par les 48 derniers acides aminés de l'isoforme jusqu'à présent connu de S100β. Il n'a pas été retrouvé la séquence correspondante à cet exon sur l'ADN génomique de souris. Par contre, celle-ci a pu être observée aussi bien chez le chimpanzé que des cercopithèques, ceux-ci exprimant par ailleurs cet exon dans des lymphocytes (figure 2a). La séquence protéique de l'isoforme normal S100β ou de l'isoforme Cog⁴⁷ sont identiques chez l'homme ou le chimpanzé. Cependant, cette séquence protéique diffère respectivement d'un acide aminé et de 6 acides aminés pour S100β et Cog⁴⁷ chez les cercopithèques. Ces données associées au fait que nous n'avons pas réussi à mettre en évidence cette séquence exonique chez les lémuriens, suggèrent que la protéine Cog⁴⁷ est récente en terme d'évolution.

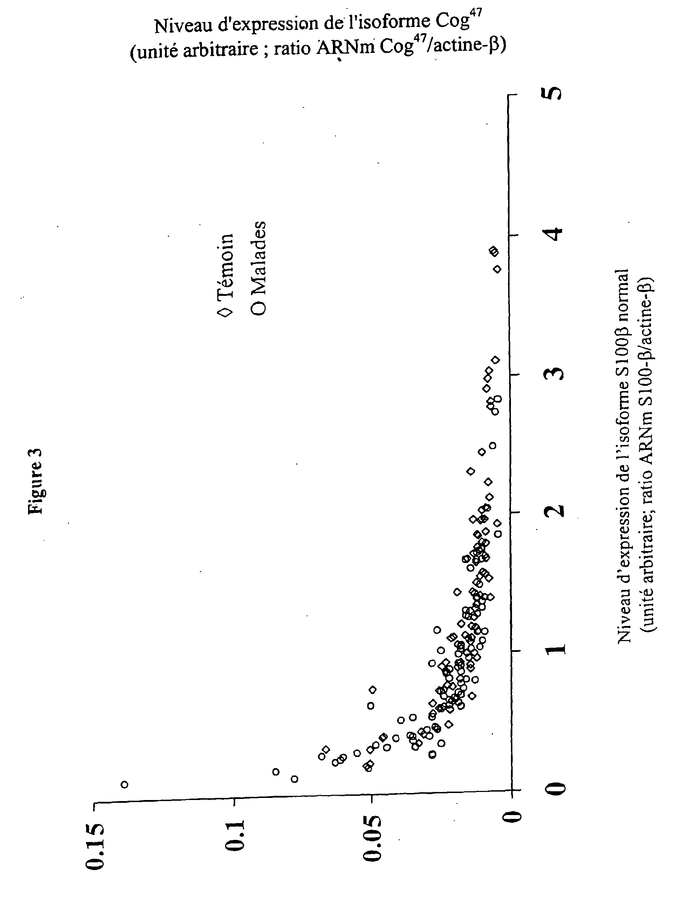

Les niveaux d'expression des isoformes de S100β et Cog⁴⁷ ont été mesurés dans le tissu cérébral de 85 patients souffrants de maladies d'Alzheimer et de 90 témoins. Par RT-PCR semi-quantitative, il a été observé que le niveau d'expression de Cog⁴⁷ semblait être inversement corrélé au niveau d'expression de S100β : plus le niveau de S100β est faible, plus le niveau de Cog⁴⁷ est élevé (Figure 3). Ce résultat suggère des mécanismes interdépendants de production de ces deux isoformes. Confortant cette possibilité, il a été également observé que le taux d'expression de S100β était diminué de plus de 37% chez les malades par rapport aux témoins, alors que celle de Cog⁴⁷ était augmentée de près de 41% chez ces mêmes malades (Tableau 1).

**Tableau 1 : Niveau d'expression des différents ARNm issus du gène S100β dans la population témoin et la population souffrant de maladie d'Alzheimer.**

| | Témoins | Malades | | P |
|---|---|---|---|---|
| n | 90 | 84 | | |
| ARNm S100β normal | 1.19 [0.63-2.24] | 0.74 [0.38-1.47] | -37.2% | 0.0001 |
| ARNm Cog⁴⁷ | 0.015 [0.008-0.026] | 0.021 [0.011-0.040] | +40.9% | 0.0004 |
| Ratio Cog⁴⁷/S100β | 0.012 [0.004-0.041] | 0.028 [0.007-0.104] | +124.3% | 0.0001 |

Les individus présentant un taux élevé d'expression de Cog⁴⁷ ont 3 fois plus de risque de développer une maladie d'Alzheimer que ceux présentant un niveau plus faible d'expression de cet isoforme (Tableau 2).

**Tableau 2 : Estimation du risque de développer une maladie d'Alzheimer en fonction des taux d'ARNm de S100β normal et de Cog⁴⁷ dans le lobe frontal.**

| | OR | IC 95% | p |
|---|---|---|---|
| Niveau élevé ARNm S100β normal | 0.33 | 0.18-0.63 | 0.0006 |
| Niveau élevé ARNm Cog⁴⁷ | 3.11 | 1.67-5.77 | 0.0003 |
| Niveau élevé Ratio Cog⁴⁷/S100β | 3.44 | 1.84-6.41 | 0.0001 |

| | | | |
|---|---|---|---|
| (valeur médiane utilisée pour différencier les niveaux élevés et faible d'expression). | | | |

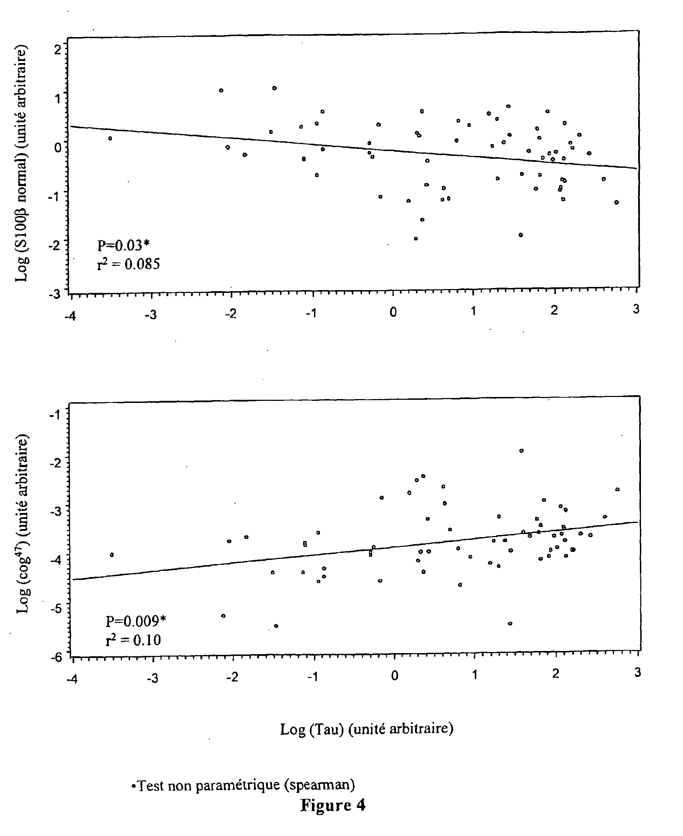

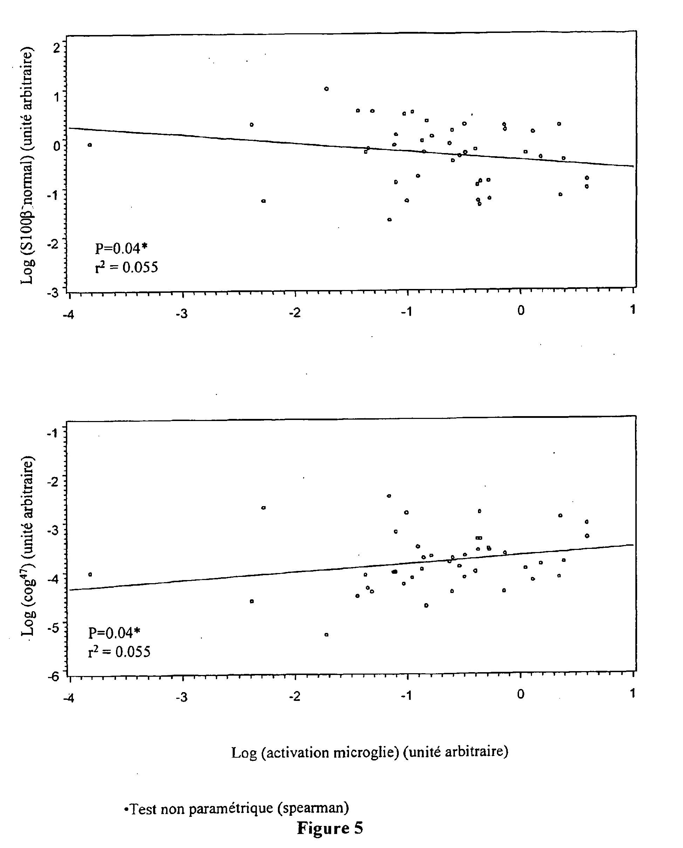

De façon cohérente à ces résultats, il a été mis en évidence une corrélation positive entre le niveau d'expression de Cog⁴⁷ et le niveau de dégénérescence neurofibrillaire (Figure 4) ou d'inflammation de la microglie (Figure 5) dans le tissu cérébral des malades, ce niveau d'expression expliquant respectivement 10 % et 5,5 % de la variance de ces marqueurs pathologiques.

Toutes les données qui précèdent semblent donc indiquer qu'outre le fait que les niveaux d'expression de S100β et de Cog⁴⁷ soient des marqueurs de la maladie d'Alzheimer, Cog⁴⁷ est un déterminant de la pathologie.

Il est apparu que des variabilités génétiques contrôlant les niveaux d'expressions de Cog⁴⁷ et/ou S100β étaient associées au déclin cognitif et au risque de développer une démence telle que la maladie d'Alzheimer. Cette hypothèse a été confirmée par une recherche systématique de polymorphismes par D-HPLC puis séquençage, réalisés sur le promoteur, les exons ainsi que l'intron 3 (englobant le nouvel exon caractéristique de Cog⁴⁷) du gène S100β.

Il a été ainsi confirmé l'existence d'un certain nombre de polymorphismes et découverts l'existence de nouveaux polymorphismes (tableau 3).

**Tableau 3 : Polymorphismes caractérisés par D-HPLC et séquençage dans le gène S100β**

| **numéro** | **position** | **localisation** | **changement^{a}** | **Ref** | **fréquence** |
|---|---|---|---|---|---|
| 1 | -1358 | promoteur | C/T | rs3788266 | 47.1% |
| 2 | -1132 | promoteur | T/C | unknown | <1% |
| 3 | -949 | promoteur | G/C | rs283965 | 43.0% |
| 4 | -797 | promoteur | G/A | rs2839364 | 12.1% |
| 5 | -761 | promoteur | C/T | unknown | |
| 6 | -738 | promoteur | A/. | unknown | 12.3% |
| 7 | -100 | promoteur | T/C | unknown | 50.0%^{b} |
| 8 | +2621 | intron | G/T | rs2186358 | 18.6% |
| 9 | +2766 | exon 2 | G/C | rs1051169 | 28.6%^{c} |
| 10 | +2821 | intron | T/C | unknown | 3.4% |
| 11 | +2852 | intron | A/G | rs2839356 | 9.2% |
| 12 | +2963 | intron | G/A | rs2839355 | 20.3%^{c} |
| 13 | +3029 | intron | T/A | unknown | 37.4% |
| 14 | +3055 | intron | G/A | unknown | <1% |
| 15 | +3492 | intron | G/A | rs6518303 | 13.2% |
| 16 | +3562 | intron | G/A | rs2300405 | 33.6% |
| 17 | +3916 | intron | C/T | rs2300404 | 35.8% |
| 18 | +3942 | intron | A/G | rs2300403 | 27.4%^{c} |
| 19 | +4416 | intron | G/A | unknown | |
| 20 | +4631 | intron | C/T | unknown | <1% |
| 21 | +5059 | intron | A/G | rs2239575 | 9.6% |
| 22 | +5128 | intron | C/T | rs881827 | 32.1% |
| 23 | +5185 | intron | G/A | rs2239574 | 34.9% |
| 24 | +5757 | 3'-UTR | C/T | rs9722 | 13.1% |
| 25 | +5759 | 3'-UTR | G/C | unknown | <1% |

| | | | | | |
|---|---|---|---|---|---|
| ^{a} les changements de base représentatif de chaque polymorphisme sont indiqués en prenant comme référence le brin codant. ^{b} pas en équilibre de Hardy-Weinberg (génotypage par digestion enzymatique et séquençage) ^{c} associé au déclin cognitif dans un sous goupe de la population ELDNOR présentant un MMS supérieur ou égal à 25. | | | | | |

A ce jour, sur les 25 polymorphismes recensés, 23 ont été étudiés dans un sous-groupe de la population ELDNOR (voir matériels et méthodes) regroupant 254 individus et présentant un MMS (minimental status) supérieur ou égal à 25. Ce premier criblage a permis de mettre en évidence trois polymorphismes (rs1051169, rs2839355 et rs2300403) localisés dans l'intron 3 ou à proximité de celui-ci, significativement associés à un déclin cognitif plus marqué.

L'étude de ces trois polymorphismes a été étendu à toute la population ELDNOR (Tableau 4). Seul deux polymorphismes rs2839355 et rs2300403, montrent alors un effet significatif et d'amplitude similaire. Ainsi pour le polymorphisme rs2000403, il a été montré que les individus porteurs du génotype GG avaient un score de MMS de 1,7 point inférieur aux individus porteurs d'au moins un allèle A (Tableau 4).

**Tableau 4 : Impact des polymorphismes rs1051169, rs2839355 et rs2000403 sur le déclin cognitif dans la population ELDNOR**

| rs1051169 | | | rs2839355 | | | Rs2300403 | | |
|---|---|---|---|---|---|---|---|---|
| | n | MMS | | n | MMS | | n | MMS |
| CC+CG | 712 | 21.3 ± 5.1 | GG+AG | 742 | 21.3 ± 5.1 | AA+AG | 700 | 21.4 ± 5.1 |
| GG | 71 | 20.2 ± 5.0 | AA | 41 | 19.7 ± 5.0 | GG | 83 | 19.7 ± 4.9 |
| Δ MMS | | - 1.1 | | | - 1.6 | | | -1.7 |
| P | | NS | P | | 0.05 | P | | 0.004 |

Cependant puisque ces deux polymorphismes sont en déséquilibre de liaison complet et pour des raisons de puissance statistique, nous avons choisi de restreindre les études suivantes au polymorphisme rs2300403.

Ainsi, dans la population de Limeil-Brévannes (voir matériel et méthode), le génotype GG du polymorphisme rs2300403 est associé à une augmentation de plus de 4 fois du risque de présenter un déclin cognitif (p=0.003 ; Tableau 5b).

**Tableau 5 : Distribution génotypique du polymorphisme rs2000403 dans des populations de patients souffrant de démences ou présentant un déclin cognitif et de témoins. (a) population ELDNOR; (b) population Limeil-Brévannes (LV); (c) combinaison des deux populations.**

| **(a) ELDNOR** | | Genotype Distribution^{b} | | | OR^{d} | |
|---|---|---|---|---|---|---|
| | n | AA | AG | GG | GG versus | p^{d} |
| | | | | | AG+AA | |
| Demented cases | 187 | 98 _{(0.52)} | 71 _{(0.38)} | 18 _{(0.10)} | 2.5 [0.9-7.2] | 0.08 |
| Controls^{b} | 145 | 77 _{(0.53)} | 63 _{(0.43)} | 5 _{(0.04)} | | |
| | | | | | | |

| **(b) LV** | | Distribution génotypique^{a} | | | OR^{d} | |
|---|---|---|---|---|---|---|
| | n | AA | AG | GG | GG versus | p^{d} |
| | | | | | AG+AA | |
| Demented cases | 125 | 62 _{(0.50)} | 42 (0.34) | 21 _{(0.17)} | 2.4 [0.9-6.5] | 0.08 |
| Cognitive décline | 36 | 15 (0.42) | 13 (0.36) | 8 (0.22) | 4.1 [1.6-10.2] | 0.003 |
| Control | 115 | 56 (0.49) | 51 (0.44) | 8 (0.07) | - | |
| | | | | | | |

| **(c) Both** | | Genotype Distribution^{c} | | | OR^{d} | |
|---|---|---|---|---|---|---|
| | n | AA | AG | GG | GG versus | p^{d} |
| | | | | | AG+AA | |
| Demented cases | 312 | 160 _{(0.51)} | 113 _{(0.36)} | 39 _{(0.13)} | 2.4 [1.2-4.7] | 0.004 |
| Controls^{b} | 260 | 133 _{(0.51)} | 114 _{(0.44)} | 13 _{(0.05)} | | |

| | | | | | | |
|---|---|---|---|---|---|---|
| ^{a} p=0.037; ^{b} p=0.08; ^{c} p=0.004; ^{d} ajusté par rapport à l'âge et au sexe | | | | | | |

En conclusion le gène S100β apparaît comme un déterminant génétique du déclin cognitif.

L'impact du polymorphisme rs2000403 sur le risque de développer une démence a également été étudié. Il a été observé que le génotype GG était associé à une augmentation du risque de développer ce type de pathologie que ce soit dans la population ELDNOR ou la population de Limeil-Brévannes. Cette augmentation n'atteint pas le seuil de signification dans ces deux populations prises séparément en raison du faible pourcentage du génotype considéré et de la taille restreinte des populations étudiées. Néanmoins, cette augmentation de risque devient significative lorsque les deux populations sont combinées ensemble (Tableau 5c ci-dessus).

Afin de conforter ce résultat, il a été étudié l'impact du polymorphisme rs2000403 sur le risque de développer une maladie d'Alzheimer. Dans la population totale (voir matériel et méthode), aucun effet significatif du génotype GG n'a été mis en évidence. Cependant, puisque les populations démentes de ELDNOR et Limeil-Brévannes sont significativement plus âgées, la population étudiée a été stratifiée en fonction de l'âge. Il est alors apparu que le génotype GG était de nouveau associé à une augmentation de plus de 2 fois du risque de développer une démence de type Alzheimer chez les plus âgés (Tableau 6).

Finalement, afin de mettre en évidence un lien potentiel entre la production de l'isoforme Cog⁴⁷ et le polymorphisme rs2000403, les échantillons cérébraux étudiés précédemment ont été génotypés pour ce polymorphisme. Dans la population témoin, nous n'avons pas pu mettre en évidence d'association entre le polymorphisme rs2000403 et la quantité d'ARNm de Cog⁴⁷. Par contre dans la population malade, nous avons observé une augmentation de 77% de la quantité d'ARNm de Cog⁴⁷ pour les individus porteurs du génotype GG par rapport à ceux porteurs d'au moins un allèle A (Tableau 7).

**Tableau 6 : Distribution génotypique du polymorphisme rs2000403 dans des populations de patients souffrant de maladies d'Alzheimer et de témoins (a) sur toute la population; (b) sur la population la plus âgée (définie par la médiane pour l'âge de la population).**

| **(a) rs2000403** | | Distribution génotypique^{a} | | | OR^{c} | |
|---|---|---|---|---|---|---|
| | n | AA | AG | GG | GG versus | p^{c} |
| | | | | | AG+AA | |
| AD cases | 608 | 285 _{(0.47)} | 261 _{(0.43)} | 62 _{(0.10)} | 1.34 [0.88-2.04] | 0.16 |
| Control | 499 | 242 _{(0.48)} | 218 _{(0.44)} | 39 _{(0.08)} | | |

| **(b) rs2000403** | | Distribution génotypique^{b} | | | OR^{c} | |
|---|---|---|---|---|---|---|
| In the oldest | n | AA | AG | GG | GG versus | p^{c} |
| | | | | | AG+AA | |
| AD cases | 284 | 129 _{(0.45)} | 122 _{(0.43)} | 33 _{(0.12)} | 2.21 [1.06-4.62] | 0.035 |
| Control | 173 | 81 _{(0.47)} | 82 _{(0.47)} | 10 _{(0.06)} | | |

| | | | | | | |
|---|---|---|---|---|---|---|
| ^{a} p=0.38; ^{b} p=0.10; ^{c} ajusté par rapport à l'âge et au sexe | | | | | | |

**Tableau 7 : Niveau d'expression des différents ARNm issus du gène S100β dans la population témoin et la population souffrant de maladie d'Alzheimer en fonction du génotype rs2000403.**

| (a) Témoins | AA+AG | GG | | p |
|---|---|---|---|---|
| n | 79 | 6 | | |
| ARNm S100β normal | 1.22 [0.63-2.36] | 1.04 [0.59-1.85] | -15.0% | ns |
| ARNm Cog⁴⁷ | 0.014 [0.008-0.026] | 0.015 [0.010-0.022] | +4.6% | ns |
| Ratio Cog⁴⁷/S100β | 0.012 [0.003-0.041] | 0.014 [0.006-0.037] | +23.0% | ns |
| | | | | |

| (b) malades | AA+AG | GG | | p |
|---|---|---|---|---|
| n | 68 | 13 | | |
| ARNm S100β normal | 0.81 [0.44-1.51] | 0.57 [0.23-1.37] | -30.6% | 0.06 |
| ARNm Cog⁴⁷ | 0.019 [0.010-0.033] | 0.033 [0.017-0.064] | +77.4% | 0.02 |
| Ratio Cog⁴⁷/S100β | 0.023 [0.007-0.076] | 0.058 [0.011-0.309] | +155.5% | 0.05 |

En résumé, nous avons observé que :
- l'ARNm issu du gène S100β subit un nouvel épissage alternatif, conduisant à la formation de l'isoforme Cog⁴⁷.
- la protéine correspondante diffère potentiellement de la forme S100β normale par les 48 derniers acides aminés.
- la quantité d'ARNm de Cog⁴⁷ est augmenté de plus de 40% dans le tissu cérébral des malades par rapport aux témoins tandis que la quantité d'ARNm de S100β est quant à elle diminuée de près de 37%.
- un taux élevé d'ARNm de Cog⁴⁷ dans le tissu cérébral est associé à une augmentation de plus de 3 fois du risque de développer une maladie d'Alzheimer.
- le niveau d'expression de Cog⁴⁷ est corrélé à la quantité de dégénérescence fibrillaire dans le tissu cérébral des malades ainsi qu'à l'activation des cellules microgliales.

Parallèlement, nous avons mis en évidence au niveau génétique que :
- le polymorphisme rs2000403, localisé dans l'intron 3 du gène S100β, est un déterminant génétique du déclin cognitif, le génotype GG étant associé à la fois à une diminution des performances cognitives mesurées par le MMS mais aussi une augmentation du risque de développer un déclin cognitif.
- le polymorphisme rs2000403 est un déterminant génétique du risque de développer une démence et en particulier une maladie d'Alzheimer, le génotype GG étant associé à une augmentation de plus de 2 fois de ce risque.
- le polymorphisme rs2000403 est associé à une augmentation de la quantité de l'ARNm de Cog⁴⁷ dans le tissu cérébral des malades, le génotype GG augmentant de 77% cette quantité par rapport aux individus non porteurs de ce génotype.

Il apparaît au vu des résultats qui précèdent que l'isoforme Cog⁴⁷ serait un déterminant du déclin cognitif et des démences, en particulier de type Alzheimer. Le polymorphisme rs2000403 en augmentant la quantité de Cog⁴⁷ accentuerait alors le risque de présenter un déclin cognitif puis une démence.

## Revendications

1. Protéine dénommée Cog⁴⁷ représentée par la séquence SEQ ID N°1 présentant une séquence d'acides aminés différente de celle de la protéine S100β normale.

2. Anticorps polyclonal ou monoclonal, ou fragment dudit anticorps, se fixant de manière spécifique sur la protéine selon la revendication 1 par affinité avec la séquence d'acides aminés différente de celle de la protéine S100β normale.

3. Utilisation d'un anticorps polyclonal ou monoclonal, ou fragment dudit anticorps, selon la revendication 2 pour la mise en oeuvre d'un test immunologique in vitro chez l'humain.

4. Utilisation d'une protéine selon la revendication 1 pour produire des anticorps se fixant de manière spécifique sur ladite protéine.

5. Procédé pour déterminer la présence d'une protéine Cog⁴⁷ représentée par la séquence SEQ ID N°1 dans un échantillon humain comprenant les étapes de :
- laisser l'échantillon à analyser réagir avec un premier anticorps selon la revendication 2,
- laisser l'échantillon à analyser réagir avec un second anticorps dirigé spécifiquement contre l'anticorps selon la revendication 2,
- laver, et
- détecter la quantité de protéine Cog⁴⁷ dans l'échantillon.

6. Procédé selon la revendication 5, **caractérisé en ce que** l'anticorps est un anticorps monoclonal.

7. Polynucléotide ARNm isolé et purifié codant pour la protéine Cog⁴⁷ représenté par la séquence SEQ ID N°2.

8. Polynucléotide ARNm d'interférence ciblant de manière spécifique l'ARNm selon la revendication 7.

9. Polynucléotide ADN capable d'exprimer le polynucléotide ARNm d'interférence selon la revendication 8 dans une cellule.

10. Utilisation du polynucléotide ARNm d'interférence selon la revendication 8 ou du polynucléotide ADN selon la revendication 9 pour la fabrication d'un médicament utile pour inhiber ou diminuer l'expression de la Protéine Cog⁴⁷.

11. Vecteur d'expression de la protéine Cog⁴⁷ comprenant au moins la séquence SEQ ID N°1 et les éléments permettant l'expression de ladite protéine dans une cellule hôte procaryote dans le but de produire une protéine recombinante.

12. Trousse de diagnostic pour déterminer la présence de la protéine Cog⁴⁷ représentée par la séquence SEQ ID N°1 dans un échantillon humain, comprenant une protéine Cog⁴⁷ selon la revendication 1, ou des fragments de ladite protéine comprenant la séquence d'acides aminés différente de celle de la protéine S100β normale, et/ou des anticorps polyclonaux ou monoclonaux, ou leurs fragments, selon la revendication 2.

13. Trousse de diagnostic selon la revendication 12, comprenant une protéine Cog⁴⁷ selon la revendication 1, et/ou des anticorps polyclonaux ou monoclonaux, ou leurs fragments, selon la revendication 2.

14. Utilisation du polynucléotide selon la revendication 7, et/ou de la protéine Cog⁴⁷ selon la revendication 1, pour la préparation d'un agent de diagnostic d'une prédisposition au déclin cognitif, à une conversion vers une démence ou à une démence déjà existante.

15. Souris transgénique comprenant dans son génome la séquence nucléotidique nécessaire pour exprimer la protéine Cog⁴⁷ représentée par la séquence SEQ ID N° 1 dans le but de développer un modèle d'étude de l'implication de Cog⁴⁷ dans des processus pathologiques et de cibles thérapeutiques potentielles.

16. Utilisation du gène S100β humain représenté par la séquence SEQ ID N°3, ou un fragment de celui-ci, pour la préparation d'un agent de diagnostic d'une prédisposition au déclin cognitif, une conversion vers une démence ou une démence déjà existante, **caractérisée en ce que** ledit gène présente au moins un polymorphisme associé à une prédisposition au déclin cognitif, à une conversion vers une démence ou à une démence déjà existante choisi parmi :
- un génotype AA au niveau du nucléotide + 2963 par rapport au site d'initiation de transcription dudit gène ; ou
- un génotype GG au niveau du nucléotide + 3942 par rapport au site d'initiation de transcription dudit gène.

17. Méthode de diagnostic in vitro de la prédisposition au déclin cognitif, à une conversion vers une démence ou à une démence déjà existante chez un humain comprenant la détermination de la présence d'un polymorphisme tel que défini dans la revendication 16 et/ou de la protéine Cog⁴⁷ selon la revendication 1.

18. Procédé d'identification *in vitro* d'une molécule capable d'interagir avec la protéine Cog⁴⁷ comprenant les étapes suivantes :
a) mettre en présence un modèle cellulaire approprié avec la protéine Cog⁴⁷ et ladite molécule à identifier pour caractériser la quantité de protéines Tau phosphorylées à l'aide d'au moins un marqueur spécifique de phosphorylation de la protéine Tau, et/ou
b) mettre en présence un modèle cellulaire approprié avec la protéine Cog⁴⁷ et ladite molécule à identifier pour caractériser le niveau d'expression d'au moins un marqueur d'inflammation de la microglie,
c) lorsque :
- la quantité de protéines Tau phosphorylées caractérisée à l'étape a) est inférieure à la quantité de protéines Tau phosphorylées caractérisée en l'absence de ladite molécule, et/ou
- le niveau d'expression dudit au moins un marqueur d'inflammation de la microglie caractérisé à l'étape b) est inférieur au niveau d'expression dudit marqueur caractérisé en l'absence de ladite molécule,
identifier ladite molécule comme étant capable d'interagir avec la protéine Cog⁴⁷.

19. Procédé d'identification selon la revendication 18, **caractérisé en ce que** le ledit au moins un marqueur spécifique de phosphorylation de la protéine Tau est un anticorps dirigé contre un épitope phosphorylé de la protéine Tau, cet épitope étant caractéristique d'une dégénérescence neuronale.

20. Procédé d'identification selon la revendication 18 ou 19, **caractérisé en ce que** ledit au moins un marqueur d'inflammation de la microglie est une cytokine.

## Claims

1. A protein named Cog¹⁷ represented by the sequence SEQ ID NO 1 having a sequence of amino acids that is different than that of the normal S100β protein.

2. A monoclonal or polyclonal antibody, or a fragment of said antibody, binding specifically to the protein according to claim 1 by affinity with the sequence of amino acids that is different than that of the normal S100β protein.

3. The use of a polyclonal or monoclonal antibody, or fragment of said antibody, according to claim 2 for the implementation of an *in vitro* immunological test in humans.

4. The use of a protein according to claim 1 for the production of antibodies that specifically bind to said protein.

5. A method for determining the presence of a Cog⁴⁷ protein represented by the sequence SEQ ID NO 1 in a human sample comprising the following steps:
- allowing the sample to be analyzed to react with a first antibody according to claim 2,
- allowing the sample to be analyzed to react with a second antibody specifically directed against the antibody according to claim 2,
- washing, and
- detecting the quantity of Cog⁴⁷ protein in the sample.

6. A method according to claim 5, wherein the antibody is a monoclonal antibody.

7. An isolated and purified mRNA polynucleotide encoding the Cog⁴⁷ protein represented by the sequence SEQ ID NO 2.

8. An interfering mRNA polynucleotide that specifically targets the mRNA according to claim 7.

9. A DNA polynucleotide capable of expressing the interfering mRNA polynucleotide according to claim 8 in a cell.

10. The use of the interfering mRNA polynucleotide according to claim 8 or of the DNA polynucleotide according to claim 9 for the manufacture of a drug useful for inhibiting or decreasing expression of the Cog⁴⁷ protein.

11. A Cog⁴⁷ protein expression vector comprising at least the sequence SEQ ID NO 1 and elements allowing the expression of said protein in a prokaryotic host cell with the purpose of producing a recombinant protein.

12. A diagnostic kit for determining the presence of the Cog⁴⁷ protein represented by the sequence SEQ ID NO 1 in a human sample, comprising a Cog⁴⁷ protein according to claim 1, or fragments of said protein comprising the amino acid sequence different than that of the normal S100β protein, and/or polyclonal or monoclonal antibodies, or fragments thereof, according to claim 2.

13. The diagnostic kit according to claim 12, comprising a Cog⁴⁷ protein according to claim 1, and/or polyclonal or monoclonal antibodies, or fragments thereof, according to claim 2.

14. The use of the polynucleotide according to claim 7, and/or the Cog⁴⁷ protein according to claim 1, for the preparation of an agent for diagnosing a predisposition to cognitive decline, to conversion to dementia or to already existing dementia.

15. A transgenic mouse comprising in its genome the nucleotide sequence required to express the Cog⁴⁷ protein represented by the sequence SEQ ID NO 1 with the purpose of developing a model for studying the involvement of Cog⁴⁷ in pathological processes and potential therapeutic targets.

16. Use of the human S100β gene represented by the sequence SEQ ID NO 3, or a fragment thereof, for the preparation of an agent for diagnosing a predisposition to cognitive decline, a conversion to dementia or an already existing dementia, wherein said gene exhibits at least one polymorphism associated with a predisposition to cognitive decline, to conversion to dementia or to already existing dementia selected from:
- an AA genotype at the nucleotide +2963 with respect to the transcription initiation site of said gene; or
- a GG genotype at the nucleotide +3942 with respect to the transcription initiation site of said gene.

17. An *in vitro* diagnostic method of the predisposition to cognitive decline, to conversion to dementia or to already existing dementia in a human comprising the determination of the presence of a polymorphism such as defined in claim 16 and/or the Cog⁴⁷ protein according to claim 1.

18. An *in vitro* method for identifying a molecule capable of interacting with the Cog⁴⁷ protein comprising the following steps:
a) bringing together a suitable cellular model with the Cog⁴⁷ protein and said molecule to be identified in order to characterize the quantity of phosphorylated tau proteins using at least one marker specific for tau protein phosphorylation, and/or
b) bringing together a suitable cellular model with the Cog⁴⁷ protein and said molecule to be identified in order to characterize the level of expression of at least one marker for microglial inflammation,
c) when:
- the quantity of phosphorylated tau proteins **characterized in** step a) is less than the quantity of phosphorylated tau proteins **characterized in** the absence of said molecule, and/or
- the level of expression of said at least one marker for microglial inflammation **characterized in** step b) is less than the level of expression of said marker **characterized in** the absence of said molecule,
identifying said molecule capable of interacting with the Cog⁴⁷ protein.

19. An identification method according to claim 18, wherein said at least one marker specific for tau protein phosphorylation is an antibody directed against a phosphorylated epitope of the tau protein, this epitope being characteristic of neuronal degeneration.

20. An identification method according to claim 18 or claim 19, wherein said at one least marker for microglial inflammation is a cytokine.

## Patentansprüche

1. Protein, bezeichnet als Cog⁴⁷, welches durch die Sequenz SEQ ID N°1 dargestellt wird und eine Aminosäuresequenz aufweist, die sich von der des normalen Proteins S100β unterscheidet.

2. Polyklonaler oder monoklonaler Antikörper oder Fragment dieses Antikörpers, welcher/welches spezifisch durch Affinität für die Aminosäuresequenz, die sich von der des normalen Proteins S100β unterscheidet, an das Protein gemäß Anspruch 1 bindet.

3. Verwendung eines polyklonalen oder monoklonalen Antikörpers oder Fragments dieses Antikörpers gemäß Anspruch 2 bei der Durchführung eines immunologischen *In-vitro*-Tests beim Menschen.

4. Verwendung eines Proteins gemäß Anspruch 1 zur Herstellung von Antikörpern, die spezifisch an dieses Protein binden.

5. Verfahren zur Bestimmung der Anwesenheit eines Cog⁴⁷-Proteins, welches durch die Sequenz SEQ ID N°1 dargestellt wird, in einer humanen Probe, umfassend die Schritte:
- Ermöglichen einer Reaktion der zu analysierenden Probe mit einem primären Antikörper gemäß Anspruch 2,
- Ermöglichen einer Reaktion der zu analysierenden Probe mit einem sekundären Antikörper, der spezifisch gegen den Antikörper gemäß Anspruch 2 gerichtet ist,
- Waschen und
- Nachweis der Menge des Cog⁴⁷-Proteins in der Probe.

6. Verfahren gemäß Anspruch 5, **dadurch gekennzeichnet, dass** der Antikörper ein monoklonaler Antikörper ist.

7. Isoliertes und gereinigtes mRNA-Polynukleotid, welches für das Protein Cog⁴⁷ kodiert und durch die SEQ ID N°2 dargestellt ist.

8. Interferenz-mRNA-Polynukleotid, welches spezifisch auf die mRNA gemäß Anspruch 7 abzielt.

9. DNA-Polynukleotid, welches das Interferenz-mRNA-Polynukleotid gemäß Anspruch 8 in einer Zelle exprimieren kann.

10. Verwendung des Interferenz-mRNA-Polynukleotids gemäß Anspruch 8 oder des DNA-Polynukleotids gemäß Anspruch 9 zur Herstellung eines Medikaments, welches zur Inhibierung oder Verminderung der Expression des Cog⁴⁷-Proteins geeignet ist.

11. Expressionsvektor des Cog⁴⁷-Proteins, umfassend wenigstens die Sequenz SEQ ID N°1 und Elemente, welche die Expression des Proteins in einer prokaryotischen Wirtszelle mit dem Ziel, ein rekombinantes Protein herzustellen, ermöglichen.

12. Diagnostik-Kit zur Bestimmung der Anwesenheit des Proteins Cog⁴⁷, welches durch die Sequenz SEQ ID N°1 dargestellt wird, in einer humanen Probe, umfassend ein Cog⁴⁷-Protein gemäß Anspruch 1 oder Fragmente dieses Proteins, welches die Aminosäuresequenz aufweist, die sich von der des normalen Proteins S100β unterscheidet, und/oder polyklonale oder monoklonale Antikörper oder Fragmente davon gemäß Anspruch 2.

13. Diagnostik-Kit gemäß Anspruch 12, umfassend ein Cog⁴⁷-Protein gemäß Anspruch 1 und/oder polyklonale oder monoklonale Antikörper oder Fragmente davon gemäß Anspruch 2.

14. Verwendung des Polynukleotids gemäß Anspruch 7 und/oder des Cog⁴⁷-Proteins gemäß Anspruch 1 zur Herstellung eines Mittels zur Diagnose einer Prädisposition für kognitiven Verfall, einer Veränderung zu einer Demenz oder einer bereits existierenden Demenz.

15. Transgene Maus, deren Genom die Nukleotidsequenz enthält, die für die Expression des Cog⁴⁷-Proteins, welches durch die SEQ ID N° 1 dargestellt wird, notwendig ist, mit dem Ziel der Entwicklung eines Modells zur Untersuchung der Rolle von Cog⁴⁷ in pathologischen Prozessen und von potentiellen therapeutischen Zielverbindungen.

16. Verwendung des humanen Gens S100β, welches durch die SEQ ID N°3 dargestellt wird, oder eines Fragments davon zur Herstellung eines Mittels zur Diagnose einer Prädisposition für kognitiven Verfall, einer Veränderung zu einer Demenz oder einer bereits existierenden Demenz, **dadurch gekennzeichnet, dass** das Gen wenigstens einen Polymorphismus aufweist, der mit einer Prädisposition für kognitiven Verfall, einer Veränderung zu einer Demenz oder einer bereits existierenden Demenz in Verbindung steht und ausgewählt ist aus:
- einem Genotyp AA auf Höhe des Nukleotids + 2963, bezogen auf die Transkriptionsinitiationsstelle des Gens, oder
- einen Genotyp GG auf Höhe des Nukleotids + 3942, bezogen auf die Transkriptionsinitiationsstelle des Gens.

17. Verfahren zur *In-vitro-*Diagnose einer Prädisposition für kognitiven Verfall, einer Veränderung zu einer Demenz oder einer bereits existierenden Demenz bei einem Menschen, welches die Bestimmung des Vorliegens eines Polymorphismus, wie in Anspruch 16 definiert, und/oder des Proteins Cog⁴⁷ gemäß Anspruch 1 umfasst.

18. Verfahren zur *In-vitro-*Identifizierung eines Moleküls, welches mit dem Cog⁴⁷-Protein interagieren kann, umfassend die folgenden Schritte:
a) Bereitstellen eines geeigneten Zellmodells mit dem Cog⁴⁷-Protein und dem zu identifizierenden Molekül zur Bestimmung der Menge an phosphorylierten Tau-Proteinen mit Hilfe wenigstens eines Markers, der für die Phosphorylierung von Tau-Protein spezifisch ist, und/oder
b) Bereitstellen eines geeigneten Zellmodells mit dem Cog⁴⁷-Protein und dem zu identifizierenden Molekül zur Bestimmung des Expressionsniveaus wenigstens eines Mikroglia-Entzündungsmarkers,
c) Identifizieren des Moleküls als fähig zur Interaktion mit dem Cog⁴⁷-Protein, wenn:
- die in Schritt a) bestimmte Menge phosphorylierter Tau-Proteine geringer ist als die Menge phosphorylierter Tau-Proteine, die in Abwesenheit des Moleküls bestimmt wurde, und/oder
- das in Schritt b) bestimmte Expressionsniveau des wenigstens einen Mikroglia-Entzündungsmarkers niedriger ist als das Expressionsniveau dieses Markers, das in Abwesenheit des Moleküls bestimmt wurde.

19. Identifikationsverfahren gemäß Anspruch 18, **dadurch gekennzeichnet, dass** der wenigstens eine Marker, der für die Phosphorylierung von Tau-Protein spezifisch ist, ein Antikörper ist, der gegen ein phosphoryliertes Epitop des Tau-Proteins gerichtet ist, wobei dieses Epitop charakteristisch für eine neuronale Degeneration ist.

20. Identifikationsverfahren gemäß Anspruch 18 oder 19, **dadurch gekennzeichnet, dass** der wenigstens eine Mikroglia-Entzündungsmarker ein Zytokin ist.
